## Europäisches Patentamt

## European Patent Office

## Office européen des brevets

(19)

(11) Veröffentlichungsnummer: **0 164 070**
**B1**

(12) **EUROPÄISCHE PATENTSCHRIFT**

(45) Veröffentlichungstag der Patentschrift: **19.09.90**

(51) Int. Cl.⁵: **C 07 D 253/08**

(21) Anmeldenummer: **85106650.6**

(22) Anmeldetag: **30.05.85**

(54) **Salze aus 3-Hydroxy-4-oxo-3,4-dihydro-1,2,3-benzotriazin und Amino-Verbindungen.**

(30) Priorität: **08.06.84 DE 3421303**

(43) Veröffentlichungstag der Anmeldung:
**11.12.85 Patentblatt 85/50**

(45) Bekanntmachung des Hinweises auf die
Patenterteilung:
**19.09.90 Patentblatt 90/38**

(84) Benannte Vertragsstaaten:
**AT BE CH DE FR GB IT LI LU NL SE**

(56) Entgegenhaltungen:
**DE-A-2 905 502**
**DE-A-3 117 985**

**CHEMICAL ABSTRACTS, Band 98, Nr.3,
17.01.1983, Columbus, Ohio, USA HARDY, PAUL
M.; LINGHAM, IAN N. "The synthesis of
peptides containing a hindered amino acid.",
Seite 544, Spalte 2, Zusammenfassung-Nr. 17
031g**

(73) Patentinhaber: **HOECHST
AKTIENGESELLSCHAFT
Postfach 80 03 20
D-6230 Frankfurt am Main 80 (DE)**

(72) Erfinder: **Volk, Alexander
Feldbergstrasse 32
D-6239 Eppstein/Taunus (DE)**
Erfinder: **König, Wolfgang, Dr.
Eppsteiner Strasse 25
D-6238 Hofheim am Taunus (DE)**

(56) Entgegenhaltungen:
**CHEMICAL ABSTRACTS, Band 96, Nr. 13,
29.03.1982, Columbus, Ohio, USA LU,
CHENGXUN; YANG, JIJIN; LENG, LIECHENG;
FENG, XINDE; LI, DECHANG. "Studies of some
bioactive N-p-methacrylamindobenzoic
esters.", Seite 342, Spalte 1,
Zusammenfassungsnr. 100 111n.**

# EP 0 164 070 B1

(56) Entgegenhaltungen:

CHEMICAL ABSTRACTS, Band 95, Nr. 8, 24.08.1981, Columbus, Ohio, USA LU, CHUN-HSUN; LIU, PEI-KANG. "Synthesis of polyamide -condensation reaction between 3,3' (isophthaloyldioxy) bis(3,4-dihydro-1,2,3-benzotriazine-4-one) and diamines under mild conditions.", Seite 3, Spalte 1, Zusammenfassung 62784d.

CHEMICAL ABSTRACTS, Band 88, Nr. 3, 16.01.1978, Columbus, Ohio, USA AHERN, T.PATRICK; NAVRATIL, THOMAS; VAUGHAN, KEITH. "A study of 3-hydroxy-1,2,3-benzotriazin-4-one and its O-methyl and O-acyl derivatives.", Seite 630, Spalte 2, Zusammenfassung 22841u.

CHEMICAL ABSTRACTS, Band 88, Nr. 15, 10. April 1978, Columbus, Ohio, USA TEETZ, V.; ECKERT, H.G.; GEIGER, R. "Synthesis and biological properties of (B1-3,5-diiodotyrosine)-insulin." Seite 612, Spalte 2, Zusammenfassung-Nr. 105 748v

**Beschreibung**

Amine werden meist in Form ihrer Salze isoliert und gereinigt. Meist werden Hydrochloride, Tosylate und von schwer zu kristallisierenden Basen auch z.B. Pikrate hergestellt. Sollen diese Salze acyliert oder alkyliert werden, müssen die Basen aus diesen Salzen wieder freigesetzt werden. Meist, löst man dieses Problem beispielsweise durch Zugabe von Basen wie tertiären Aminen zum Reaktionsansatz. Diese Basen bringen oft weitere Verunreinigungen in die Ansätze, müssen anschließend wieder entfernt werden und können zu Nebenprodukten führen. Diese Nachteile haben Salze mit 1-Hydroxybenzotriazol (HOBt) nicht (Int. J. Peptide Protein Res. *20*, 387—395 (1982)), da diese Salze mit aktivierten Estern oder mit Carbonsäuren unter Zusatz von Dicyclohexylcarbodiimid (DCC) zu den entsprechenden Amiden abreagieren. Dabei nutzt man auch noch den katalysierenden Effekt des HOBt aus, das die Acylierung der Amine mit Aktivestern beschleunigt (Chem. Ber. *106*, 3626—3635 (1973)) und die Racemisierung bei der Peptidsynthese unter Verwendung von DCC senkt (Chem. Ber. *103*, 788—798 (1970)).

Nun gibt es eine Reihe von Aminen, wie z.B. das L-Threoninol oder das L-Leucinol, deren Reinigung Schwierigkeiten macht. So wird Threoninol aus Threoninmethylester und Leucinol aus Leucin mit Reduktionsmitteln wie LiAlH$_4$ hergestellt. Die Amine fallen dabei als uneinheitliche Öle an, deren, Reinigung über die üblichen Salze nicht möglich war. Auch mit HOBt geben diese Amine keine kristallisierenden Salze.

Überraschenderweise kristallisieren diese Basen dagegen mit 3-Hydroxy-4-oxo-3,4-dihydro-1,2,3-benzotriazin (HOObt). Die Basen können auf diese Weise einfach aus Gemischen isoliert und gereinigt werden. Auch andere Amino-Verbindungen wie z.B. Aminosäureester können so gereinigt werden. Merkwürdigerweise kristallisieren ein Teil der verwendeten Amino-Verbindungen, wie z.B. Threoninol nur mit zwei Äquivalenten HOObt. Andere, wie z.B. das Leucinol oder H-Ser(Bu$^t$)-OBu$^t$ begnügen sich dagegen mit einem Moläquivalent HOObt. Amorphe HOObt-Salze, die ebenfalls zur Reinigung von Basen herangezogen werden können, zeigen meist einen schwankenden Gehalt an HOObt (zwischen 1 und 2 Moläquivalenten).

Die Erfindung betrifft somit Salze aus 3-Hydroxy-4-oxo-3,4-dihydro-1,2,3-benzotriazin (HOObt) und einem primären aliphatischen Amin, einem Ester einer Aminosäure, einem Derivat oder Abkömmling einer α-Aminosäure oder einem Derivat oder Abkömmling eines Peptids.

Diese Salze können als kristalline oder amorphe Feststoffe vorliegen. Das Molverhältnis HOObt:Monoamino-Verbindung beträgt vorzugsweise 1:1 bis 2:1. Bei Di- und Polyamino-Verbindungen ist das Verhältnis entsprechend größer.

Ein primäres aliphatisches Amin ist beispielsweise ein Alkylamin. Es kann zusätzlich eine Hydroxyfunktion oder eine Esterfunktion aufweisen.

Unter einem Derivat einer α-Aminosäure oder eines Peptids wird beispielsweise deren Ester verstanden, unter Abkömmlingen beispielsweise deren reduzierte Formen. Entsprechende natürlich vorkommende α-Aminosäuren sind z.B. in Houben-Weyl, Methoden der Organischen Chemie, Bd, XV/1 und XV/2 beschrieben.

Bevorzugte Derivate von α-Aminosäuren sind deren Amide und Ester, insbesondere die (C$_1$—C$_6$)-Alkyl-bzw. Benzylester natürlich vorkommender α-Aminosäure, worin weitere funktionelle Gruppen ggf. in an sich bekannter Weise geschützt vorliegen können. Bevorzugte Abkömmlinge von α-Aminosäuren sind solche, worin die Carboxylfunktion zur —CH$_2$OH reduziert vorliegt. Unter Peptiden, die in der oben beschriebenen Weise derivatisiert, modifiziert oder geschützt vorliegen können, versteht man vorzugsweise aus natürlich vorkommenden α-aminosäuren aufgebaute Oligopeptide.

Besonders bevorzugt sind Salze aus HOObt und H-Leu-ol, H-Thr-ol, H-Ile-ol, H-Phe-ol oder H-Thr(Bu$^t$)-ol, aus HOObt und H-Thr-OMe oder H-Ser(Bu$^t$)-OBu$^5$, sowie aus HOObt und H-Cus(SBu$^t$)-Leu-ol, H-Cys(SBu$^t$)-4-Abu-OBu$^t$ oder H-Thr-Cys(SBu$^t$)-Thr-ol.

Die Erfindung betrifft auch ein Verfahren zur Herstellung eines Salzes gemäß einem der Ansprüche 1 bis 9, das dadurch gekennzeichnet ist, daß man HOOBt zusammen mit der Amino-Verbindung in einem geeigneten Lösungsmittel löst und das Salz aus dieser Lösung

a) durch Zugabe eines weiteren Lösungsmittels, in dem das Salz weniger löslich ist, ausfällt,

b) durch Einengen abscheidet oder

c) durch Abkühlen der gesättigten Lösung abscheidet.

Die Salze werden vorzugsweise folgendermaßen herstestellt: Zunächst wird die Amino-Verbindung mit der entsprechenden Menge (1 bis 2 Äquivalente pro NH$_2$-Gruppe) HOObt z.B. in Alkoholen, vorzugsweise in Methanol gelöst.

Aus diesen Lösungen fällt man die Salze durch Zugabe von Ethern, wie z.B. Diethylether oder Diisopropylether aus, oder die Lösungen werden eingeengt und/oder abgekült und der Rückstand wird aus Ethern, wie z.B. Diethylether oder tert.-Butyl-methylether kristallisiert. Die Verunreinigungen bleiben dabei in der Regel in der Mutterlauge zurück. Die Bestimmung des HOObt-Gehalts wird über ein NMR-Spektrum bestimmt.

Die Erfindung betrifft weiterhin die Verwendung der oben genannten Slaze bei der Reinigung von Amino-Verbindungen und bei der Herstellung von Carbonsäureamiden in Gegenwart von Carbodiimiden.

Die Salze können, wie die HOBt-Salze, direkt zur Acylierung eingesetzt werden. Dabei hat das HOObt gegenüber dem HOBt noch den Vorteil, daß bei der Acylierung der Basen mit Peptiden die Racemisierung stärker gesenkt wird (Chem. Ber. *103*, 2034—2040 (1970); Chem. Ber. *103*, 2024—2033 (1970); K. Brunfeldt (Hrsg.): Peptides 1980, S. 174—179, Scriptor, Copenhagen, 1981; Int. J. Peptide Protein Res. *17*, 197—204 (1981)).

Mit Carbonsäureaktivestern, wie z.B. den 2,4,5-Trichlorphenylestern oder den 2- bzw. 4-Nitrophenylestern reagieren die HOObt-Salze ähnlich schnell ab, wie mit den HOBt-Salzen. Für die Acylierung mit Carbonsäuren verwendet man im allgemeinen Carbodiimide. Besonders bevorzugt ist DCC. Als Lösungsmittel für die Acylierung dieser Salze sind stark polare Lösungsmittel, wie z.B. Dimethylformamid oder Dimethylacetamid bestens geeignet. Das HOObt kann nach der Umsetzung durch Extraktion mit NaHCO$_3$-Lösungen entfernt werden.

Die Folgenden Beispiele dienen zur Erläuterung der Erfindung, ohne diese darauf zu beschränken.

Beispiel 1

L-Leucinol · HOObt (H-Leu-ol-HOObt)

Zu 250 ml auf −5°C gekühltes Tetrahydrofuran gibt man unter Rühren 11,4 g LiAlH$_4$. Zu dieser Suspension gibt man unter Rühren innerhalb von 45 Minuten 28,86 g (0,22 Mol) L-Leucin. Anschließend kocht man 4 Stunden am Rückfluß. Danach kühlt man auf 0°C ab, gibt 250 ml Diethylether zu und läßt unter Rühren langsam 50 ml Wasser zutropfen. Der Niederschlag wird abgesaugt und der Rückstand zweimal mit je 150 ml Methanol ausgekocht und heiß abgesaugt. Die vereinigten Filtrate werden eingeengt. Der Rückstand wird in 100 ml abs. Ethanol aufgenommen und mit 200 ml Ether versetzt. Man läßt über Nacht bei 4°C stehen, saugt den Niederschlag ab un engt das Filtrat ein. Das resultierende Öl (26,1 g) wird in 150 ml Methanol gelöst. Dazu gibt man 36 g (0,22 Mol) HOObt und 200 ml Diethylether und läßt das Salz bei 4°C auskristallisieren. Der Niederschlag wird abgesaugt und mit Ether gewaschen.

Ausbeute: 44,5 g blaßgelbe Kristalle (72%), Schmp. 158°—160°C (unter Zersetzung), $[\alpha]_D^{23}$=+6,5°C (c=1, in Methanol).

Analog wurden folgende Salze hergestellt, wobei in den Fällen, in denen die Amino-Verbindungen mit 2 Mol HOObt kristallisieren, die doppelte Menge an HOObt zugesetzt wird:

| | Schmp. | $[\alpha]_D^{23}$ (c=1, in Methanol) |
|---|---|---|
| H-Thr(Bu$^t$)-ol · 2HOObt (aus H-Thr(Bu$^t$)-OH) | 147°C | −8,2° |
| H-Ile-ol · HOObt (aus H-Ile-OH) | 178—179°C | +8,8° |
| H-Thr-ol · 2HOObt (aus H-Thr-OMe) | 157°C | −5,7° |
| H-Phe-ol · HOObt (aus H-Phe-OH) | 150—152°C | −0,5° |

Beispiel 2

10 mMol einer Amino-Verbindung werden zusammen mit 1,63 g (10 mMol) HOObt in 20 ml Methanol gelöst. Diese Lösung wird mit 40 ml Diethylether versetzt. Man läßt bei 4°C das Salz auskristallisieren, saugt ab, wäscht mit Ether nach und trocknet im Vakuum.

| | Schmp. | $[\alpha]_D^{23}$ (c=1, Methanol) | Ausbeute |
|---|---|---|---|
| H-Thr-OMe · HOObt | 169—170°C | −4,7° | 90% |
| H-Ser(Bu$^t$)-OBu$^t$ · HOObt | 128°C | 0° | 85% |

Beispiel 3

Zu einer Lösung von 10 mMol eines Fmoc-Peptids in 50 ml Dimethylformamid gibt man 10,5 ml (100 mMol) Diethylamin. Nach 5 Min Rühren bei Raumtemperatur wird eingeengt. Der Rückstand wird in 30 ml Methanol zusammen mit 1,65 g (10 mMol) bzw. 3,3 g (20 mMol) HOObt gelöst. Die Lösung wird eingeengt und der Rückstand mit Ether verrieben. Der Niederschlag wird abgesaugt und mit Ether gewaschen. Der Gehalt an HOObt schwankt etwas je nach Zugabe von HOObt und Ansatz. Obwohl die Salze nicht in konstanten Molverhältnissen kristallisieren, ist durch Dünnschichtchromatographie ein deutlicher Reinigungseffekt nachweisbar.

|  | Schmp. | $[\alpha]_D^{23}$ (c=1, Methanol) |
|---|---|---|
| H-Cys(SBu$^t$)-Thr-ol · 1,5HOObt | amorph | −26,3° |
| H-Cys(SBu$^t$)-Leu-ol · HOObt | 155°C | −45,3° |
| H-Cys(SBu$^t$)-4-Abu-OBu$^t$ · 2HOObt | 109—111°C | −5,7° |
| H-Thr-Cys(SBu$^t$)-Thr-ol · 1,75HOObt | 140°C (Zers.) | −55,7° |

Beispiel 4

Fmoc-Cys(SBu$^t$)-Thr-ol

Zu einer Lösung von 8,62 g (20 mMol) H-Thr-ol · 2HOObt in 50 ml Dimethylformamid gibt man 12,2 g (20 mMol) Fmoc-Cys(SBu$^t$)-OTcp. Man läßt über Nacht stehen, engt ein und verteilt den Rückstand zwischen Essigester und 50 ml einer gesättigten NaHCO$_3$-Lösung. Anschließend wird die Essigester-Phase mit einem KHSO$_4$/K$_2$SO$_4$-Puffer und Wasser ausgeschüttelt, über Na$_2$SO$_4$ getrocknet und eingeengt. Die Substanz wird aus Diethylether kristallisiert.

Ausbeute 70%, Schmp. 155°C (Zers.), $[\alpha]_D^{23}$=−86,5° (c=1, Methanol).

Analog wurden hergestellt:

|  | Ausbeute | Schmp. | $[\alpha]_D^{23}$ (c=1, Methanol) |
|---|---|---|---|
| Fmoc-Cys(SBu$^t$)-Thr(Bu$^t$)-ol (Reinigung durch Chromatographie auf Kieselgel in Methylenchlorid) | 75% | amorph | −69,9° |
| Fmoc-Cys(SBu$^t$)-Leu-ol | 67% | 149°C (Zers.) | −82,8° |

Beispiel 5

Fmoc-Ser(Bu$^t$)-Cys(SBu$^t$)-Leu-ol

Zu einer Lösung von 2,49 g (6,5 mMol) Fmoc-Ser(Bu$^t$)-OH und 3,06 g H-Cys(SBu$^t$)-Leu-ol · HOObt in 25 ml Dimethylacetamid gibt man bei 0°C 1,44 g (7 mMol) DCC. Man läßt zunächst 1 Stunde bei 0°C rühren und dann über Nacht bei Raumtemperatur stehen. Der Niederschlag wird abgesaugt und das Filtrat eingeengt und wie bei Beispiel 4 aufgearbeitet.

Ausbeute 3,1 g (71%), Schmp. 144°C, $[\alpha]_D^{22}$=−76,6° (c=1, Methanol).

Analog wurden hergestellt:

|  | Ausbeute | Schmp. | $[\alpha]_D^{23}$ (c=1) |
|---|---|---|---|
| Fmoc-Thr(Bu$^t$)-Cys(SBu$^t$)-Thr-ol (Reinigung durch Chromatographie auf Kieselgel in Methylenchlorid/ Methanol wie 9,2:0,8) | 72% | amorph | −46,2° (in Methanol) |
| Fmoc-His-Cys(SBu$^t$)-Thr-ol | 80% | 172°C | −49,2° (in 90% Essigsäure) |
| Fmoc-Thr-Cys(SBu$^t$)-Thr-ol | 95% | 175°C | −90,5° (in Methanol) |
| Fmoc-Tyr(Et)-Cys(SBu$^t$)-Thr-ol | 83% | 126°C | −37,4° (in Methanol) |

**Patentansprüche für die Vertragsstaaten: BE, CH, DE, FR, GB, IT, LU, NL, SE**

1. Salz aus 3-Hydroxy-4-oxo-3,4-dihydro-1,2,3-benzotriazin (HOObt) und einem primären aliphatischen Amin, einem Ester einer Aminosäure, einem Derivat oder Abkömmling einer α-Aminosäure oder einem Derivat oder Abkömmling eines Peptids.

2. Salz gemäß Anspruch 1, dadurch gekennzeichnet, daß es als Feststoff vorliegt.

3. Salz gemäß Anspruch 1 und/oder 2, dadurch gekennzeichnet, daß das Molverhältnis HOObt:Monoamino-Verbindung=1:1 bis 2:1 beträgt.

4. Salz gemäß einem oder mehreren der Ansprüche 1 bis 3, dadurch gekennzeichnet, daß es aus HOObt und Amiden oder Estern, insbesondere ($C_1$—$C_6$)-Alkyl- bzw. Benzylester, natürlich vorkommender α-Aminosäuren besteht.

5. Salz gemäß einem oder mehreren der Ansprüche 1 bis 3, dadurch gekennzeichnet, daß es aus HOObt und einem Abkömmling einer α-Aminosäure, worin die Carboxylfunktion zu —$CH_2OH$ reduziert vorliegt, besteht.

6. Salz gemäß einem oder mehreren der Ansprüche 1 bis 3 und 5, dadurch gekennzeichnet, daß es aus HOObt und H-Leu-ol, H-Thr-ol, H-Ile-ol, H-Phe-ol oder H-Thr(Bu$^t$)-ol besteht.

7. Salz gemäß einem oder mehreren der Ansprüche 1 bis 4, dadurch gekennzeichnet, daß es aus HOObt und H-Thr-OMe oder H-Ser(Bu$^t$)-OBu$^t$ besteht.

8. Salz gemäß einem oder mehreren der Ansprüche 1 bis 3, dadurch gekennzeichnet, daß es aus HOObt und H-Cys(SBu$^t$)-Thr-ol, H-Cys(SBu$^t$)-Leu-ol, H-Cys(SBu$^t$)-4-Abu-OBu$^t$ oder H-Thr-Cys(SBu$^t$)-Thr-ol besteht.

9. Verfahren zur Herstellung eines Salzes gemäß einem der Ansprüche 1 bis 8, dadurch gekennzeichnet, daß man HOObt zusammen mit der Amino-Verbindung in einem geeigneten Lösungsmittel löst und das Salz aus dieser Lösung

a) durch Zugabe eines weiteren Lösungsmittels, in dem das Salz weniger löslich ist, ausfällt,

b) durch Einengen abscheidet oder

c) durch Abkühlen der gesättigten Lösung abscheidet.

10. Verwendet eines Salzes gemäß einem oder mehreren der Ansprüche 1 bis 8 bei der Reinigung der entsprechenden Aminverbindungen.


## Patentansprüche für den Vertragsstaat: AT

1. Verfahren zur Herstellung eines Salzes aus 3-Hydroxy-4-oxo-3,4-dihydro-1,2,3-benzotriazin (HOObt) und einem primären aliphatischen Amin, einem Ester einer Aminosäure, einem Derivat oder Abkömmling einer α-Aminosäure oder einem Derivat oder Abkömmling eines Peptids, dadurch gekennzeichnet, daß man HOObt zusammen mit der Amino-Verbindung in einem geeigneten Lösungsmittel löst und das Salz aus dieser Lösung

a) durch Zugabe eines weiteren Lösungsmittels, in dem das Salz weniger löslich ist, ausfällt,

b) durch Einengen abscheidet oder

c) durch Abkühlen der gesättigten Lösung abscheidet.

2. Verfahren gemäß Anspruch 1, dadurch gekennzeichnet, daß das hergestellte Salz als Feststoff vorliegt.

3. Verfahren gemäß Anspruch 1 und/oder 2, dadurch gekennzeichnet, daß das Molverhältnis HOObt:Monoamino-Verbindung=1:1 bis 2:1 beträgt.

4. Verfahren gemäß einem oder mehreren der Ansprüche 1 bis 3, dadurch gekennzeichnet, daß das Salz aus HOObt und Amiden oder Estern, insbesondere ($C_1$—$C_6$)-Alkyl- bzw. Benzylester, natürlich vorkommender α-Aminosäuren besteht.

5. Verfahren gemäß einem oder mehreren der Ansprüche 1 bis 3, dadurch gekennzeichnet, daß das Salz aus HOObt und einem Abkömmling einer α-Aminosäure, worin die Carboxylfunktion zu —$CH_2OH$ reduziert vorliegt, besteht.

6. Verfahren gemäß einem oder mehreren der Ansprüche 1 bis 3 und 5, dadurch gekennzeichnet, daß das Salz aus HOObt und H-Leu-ol-, H-Thr-ol, H-Ile-ol, H-Phe-ol oder H-Thr(Bu$^t$)-ol besteht.

7. Verfahren gemäß einem oder mehreren der Ansprüche 1 bis 4, dadurch gekennzeichnet, daß das Salz aus HOObt und H-Thr-OMe oder H-Ser(Bu$^t$)-OBu$^t$ besteht.

8. Verfharen gemäß einem oder mehreren der Ansprüche 1 bis 3, dadurch gekennzeichnet, daß das Salz aus HOObt und H-Cys(SBu$^t$)-Thr-ol, H-Cys(SBu$^t$)-Leu-ol, H-Cys(SBu$^t$)-4-Abu-OBu$^t$ oder H-Thr-Cys(SBu$^t$)-Thr-ol besteht.

9. Verwendung eines gemäß einem oder mehreren der Ansprüche 1 bis 8 hergestellten Salzes bei der Reinigung der entsprechenden Aminverbindungen.


## Revendications pour les Etats Contractants: BE, CH, DE, FR, GB, IT, LU, NL, SE

1. Sel de 3-hydroxy-4-oxo-3,4-dihydro-1,2,3-benzotriazine (HOObt) et d'une amine primaire aliphatique, d'un ester d'un aminoacide, d'un dérivé ou d'une modification d'un α-aminoacide ou d'un dérivé ou d'une modification d'un peptide.

2. Sel selon la revendication 1, caractérisé en ce qu'il se présente sous forme d'un solide.

3. Sel selon la revendication 1 et/ou 2, caractérisé en ce que le rapport molaire HOObt:composé mono-amino va de 1:1 à 2:1.

4. Sel selon une ou plusieurs des revendications 1 à 3, caractérisé en ce qu'il est constitué de HOObt et d'amides ou d'esters, en particulier d'esters alkyliques en $C_1$—$C_6$ ou benzyliques, d'α-aminoacides existant dans la nature.

5. Sel selon une ou plusieurs des revendications 1 à 3, caractérisé en ce qu'il est constitué de HOObt et d'une modification d'un α-aminoacide, dans laquelle la fonction carboxy est réduite en —$CH_2OH$.

6. Sel selon une ou plusieurs des revendications 1 à 3 et 5, caractérisé en ce qu'il est constitué de HOObt et de H-Leu-ol, H-Thr-ol, H-Ile-ol, H-Phe-ol ou H-Thr(Bu$^t$)-ol.

7. Sel selon une ou plusieurs des revendications 1 à 4, caractérisé en ce qu'il est constitué de HOObt et de H-Thr-OMe ou H-Ser(Bu$^t$)-OBu$^t$.

8. Sel selon une ou plusieurs des revendications 1 à 3, caractérisé en ce qu-il est constitué de HOObt et de H-Cys(SBu$^t$)-Thr-ol, H-Cys(SBu$^t$)-Leu-ol, H-Cys(SBu$^t$)-4-Abu-OBu$^t$ ou de H-Cys(SBu$^t$)-Thr-ol.

9. Procédé pour la préparation d'un sel selon l'une des revendications 1 à 8, caractérisé en ce que l'on dissout de la HOObt conjointement avec le composé aminé dans un solvant approprié, et, à partir de cette solution,

a) on fait précipiter le sel par addition d'un autre solvant dans lequel le sel est moins soluble,

b) on sépare le sel par concentration ou

c) on sépare le sel par refroidissement de la solution saturée.

10. Utilisation d'un sel selon une ou plusieurs des revendications 1 à 8, dans la purification des composés aminés correspondants.

**Revendications pour l'Etat Contractant: AT**

1. Procédé pour la préparation d'un sel de 3-hydroxy-4-oxo-3,4-dihydro-1,2,3-benzotriazine (HOObt) et d'une amine primaire aliphatique, d'un ester d'un aminoacide, d'un dérivé ou d'une modification d'un α-aminoacide ou d'un dérivé ou d'une modification d'un peptide, caractérisé en ce que l'on dissout de la HOObt conjointement avec le composé aminique dans un solvant approprié, et, à partir de cette solution,

a) on fait précipiter le sel par addition d'un autre solvant dans lequel le sel est moins soluble,

b) on sépare le sel par concentration ou

c) on sépare le sel par refroidissement de la solution saturée.

2. Procédé selon la revendication 1, caractérisé en ce que le sel préparé se trouve sous forme d'un solide.

3. Procédé selon la revendication 1 et/ou 2, caractérisé en ce que le rapport molaire HOObt:composé mono-amino va de 1:1 à 2:1.

4. Procédé selon une ou plusieurs des revendications 1 à 3, caractérisé en ce que le sel est constitué de HOObt et d'amides ou d'esters, en particulier d'esters alkyliques en $C_1$—$C_6$ ou benzyliques, d'α-aminoacides existant dans la nature.

5. Procédé selon une ou plusieurs des revendications 1 à 3, caractérisé en ce que le sel est constitué de HOObt et d'une modification d'un α-aminoacide, dans laquelle la fonction carboxy est réduite en —$CH_2OH$.

6. Procédé selon une ou plusieurs des revendications 1 à 3 et 5, caractérisé en cs que le sel est constitué de HOObt et de H-Leu-ol, H-Thr-ol, H-Ile-ol, H-Phe-ol ou H-Thr(Bu$^t$)-ol.

7. Procédé selon une ou plusieurs des revendications 1 à 4, caractérisé en ce que le sel est constitué de HOObt et de H-Thr-OMe ou H-Ser(Bu$^t$)-OBu$^t$.

8. Procédé selon une ou plusieurs des revendications 1 à 3, caractérisé en ce que le sel est constitué de HOObt et de H-Cys(SBu$^t$)-Thr-ol, H-Cys(SBu$^t$)-Leu-ol, H-Cys(SBu$^t$)-4-Abu-OBu$^t$ ou H-Cys(SBu$^t$)-Thr-ol.

9. Utilisation d'un sel préparé selon une ou plusieurs des revendications 1 à 8, dans la purification des composés aminés correspondants.

**Claims for the Contracting States: BE, CH, DE, FR, GB, IT, LU, NL, SE**

1. A salt composed of 3-hydroxy-4-oxy-3,4-dihydro-1,2,3-benzotriazine (HOObt) and of a primary aliphatic amine, of an ester of an amino acid, of a derivative or modified form of an α-amino acid or of a derivative or modified form of a peptide.

2. A salt as claimed in claim 1, which is in the form of a solid.

3. A salt as claimed in claim 1 and/or 2, in which the mola ratio of HOObt:monoamino compound is 1:1 to 2:1.

4. A salt as claimed in one or more of claims 1 to 3, which is composed of HOObt and amides or esters, in particular ($C_1$—$C_6$)-alkyl or benzyl esters, of naturally occurring α-amino acids.

5. A salt as claimed in one or more of claims 1 to 3, which is composed of HOObt and of a modified form of an α-amino acid in which the carboxyl group has been reduced to —$CH_2OH$.

6. A salt as claimed in one or more of claims 1 to 3 and 5, which is composed of HOObt and H-Leu-ol, H-Thr-ol, H-Ile-ol, H-Phe-ol or H-Thr(Bu$^t$)-ol.

7. A salt as claimed in one or more of claims 1 to 4, which is composed of HOObt and H-Thr-OMe or H-Ser(Bu$^t$)-OBu$^t$.

8. A salt as claimed in one or more of claims 1 to 3, which is composed of HOObt and H-Cys(SBu$^t$)-Thr-ol, H-Cys(SBu$^t$)-Leu-ol, H-Cys(SBu$^t$)-4-Abu-OBu$^t$ or H-Thr-Cys(SBu$^t$)-Thr-ol.

7

9. A process for the preparation of a salt as claimed in any of claims 1 to 8, which comprises HOObt being dissolved together with the amino compound in a suitable solvent, and from this solution the salt being

a) precipitated by addition of another solvent in which the salt is less soluble,

b) separated out by concentration or

c) separated out by cooling the saturated solution.

10. The use of a salt as claimed in one or more of claims 1 to 8 for the purification of the corresponding amine compounds.

**Claims for the Contracting State: AT**

1. A process for the preparation of a salt composed of 3-hydroxy-4-oxo-3,4-dihydro-1,2,3-benzotriazine (HOObt) and of a primary aliphatic amine, of an ester of an amino acid, of a derivative or modified form of an α-amino acid or of a derivative or modified form of a peptide, which comprises HOObt being dissolved together with the amino compound in a suitable solvent, and from this solution the salt being

a) precipitated by addition of another solvent in which the salt is less soluble,

b) separated out by concentration or

c) separated out by cooling the saturated solution.

2. The process as claimed in claim 1, wherein the prepared salt is in the form of a solid.

3. The process as claimed in claim 1 and/or 2, in which the molar ratio of HOObt to monoamino compound is 1:1 to 2:1.

4. The process as claimed in one or more of claims 1 to 3, wherein the salt is composed of HOObt and amides or esters, in particular $(C_1—C_6)$-alkyl or benzyl esters, of naturally occurring α-amino acids.

5. The process as claimed in one or more of claims 1 to 3, wherein the salt is composed of HOObt and of a modified form of an α-amino acid in which the carboxyl group has been reduced to —$CH_2OH$.

6. The process as claimed in one or more of claims 1 to 3 and 5, wherein the salt is composed of HOObt and H-Leu-ol, H-Thr-ol, H-Ile-ol, H-Phe-ol or H-Thr(Bu$^t$)-ol.

7. The process as claimed in one or more of claims 1 to 4, wherein the salt is composed of HOObt and H-Thr-OMe or H-Ser(Bu$^t$)-OBu$^t$.

8. The process as claimed in one or more of claims 1 to 3, wherein the salt is composed of HOObt and H-Cys(SBu$^t$)-Thr-ol, H-Cys(SBu$^t$)-Leu-ol, H-Cys(SBu$^t$)-4-Abu-OBu$^t$ or H-Thr-Cys(SBu$^t$)-Thr-ol.

9. The use of a salt prepared as claimed in one or more of claims 1 to 8 for the purification of the corresponding amine compounds.